# FASCICULE DE BREVET EUROPEEN

(11) **EP 0 875 579 B1**
(45) Date de publication et mention de la délivrance du brevet: **27.06.2007**
(21) Numéro de dépôt: 97201337.9
(22) Date de dépôt: 03.05.1997
(51) Int. Cl.: C12N 15/74, C12N 15/70, C12P 7/56, C12N 9/04, A23K 1/00, C12N 1/20

(54) **Production de L(+)-Lactate**
Herstellung von L(+)-Lactat
Production of L(+)lactate

(43) Date de publication de la demande: 04.11.1998
(73) Titulaire: SOCIETE DES PRODUITS NESTLE S.A., 1800 Vevey (CH)
(72) Inventeur: Lapierre, Luciane, 1616 Attalens (CH); Germond, Jacques Edouard, 1023 Crissier (CH); Pridmore, David, Vers-chez-les Blanc, 1000 Lausanne 26 (CH); Mollet, Beat, 1012 Lausanne (CH); Delley, Michèle, 1010 Lausanne (CH)
(74) Mandataire: Dixon, Sarah

(56) Documents cités:
- EP-A- 0 577 904
- EP-A- 0 638 642
- US-A- 5 416 020
- CHEMICAL ABSTRACTS, vol. 121, no. 13, 26 septembre 1994 Columbus, Ohio, US; abstract no. 150201, BHOWMIK, T. ET AL: "Cloning, characterization and insertional inactivation of the Lactobacillus helveticus D- lactate dehydrogenase gene" XP002043386 & APPL. MICROBIOL. BIOTECHNOL. (1994), 41(4), 432-9 CODEN: AMBIDG;ISSN: 0175-7598,
- TAGUCHI, HAYAO ET AL: "Essential role of arginine 235 in the substrate-binding of Lactobacillus plantarum D- lactate dehydrogenase" J. BIOCHEM. (TOKYO) (1994), 115(5), 930-6 CODEN: JOBIAO;ISSN: 0021-924X, XP002043384
- KOCHHAR, SUNIL ET AL: "Primary structure, physicochemical properties, and chemical modification of NAD+-dependent D- lactate dehydrogenase. Evidence for the presence of Arg-235, His-303, Tyr-101, and Trp-19 at or near the active site" J. BIOL. CHEM. (1992), 267(12), 8499-513 CODEN: JBCHA3;ISSN: 0021-9258, XP002043385
- CHEMICAL ABSTRACTS, vol. 119, no. 19, 8 novembre 1993 Columbus, Ohio, US; abstract no. 199233, DELCOUR, J. ET AL: "Molecular genetics of lactate dehydrogenases from lactic acid bacteria" XP002043387 & LAIT (1993), 73(2), 127-31 CODEN: LAITAG;ISSN: 0023-7302,
- FERAIN T ET AL: "LACTOBACILLUS PLANTARUM LDHL GENE: OVEREXPRESSION AND DELETION" JOURNAL OF BACTERIOLOGY, vol. 176, no. 3, pages 596-601, XP000608378

## Description

La présente invention a pour objet des souches bactériennes recombinées génétiquement ainsi qu'un procédé de production de ces souches.

### ETAT DE LA TECHNIQUE:

La fermentation est une dégradation d'une source de carbone au cours de laquelle l'accepteur final d'hydrogène est un composé organique. Par voie fermentaire lactique, certaines souches bactériennes produisent un mélange racémique des deux formes isomériques du lactate, le D(-)-lactate et le L(+)-lactate, pour la régénération du NAD⁺, par réduction du pyruvate par deux lactate-deshydrogénases NAD-dépendantes spécifiques.

Il est connu que certains individus présentent une intolérance à la réduction du lactose. Cette mauvaise digestion du lactose est souvent dûe à l'absence d'une quantité suffisante de β-galactosidase dans le petit intestin. Différentes études (Kolars et al, N Engl J Med, 310: 1-3, 1984; Marteau et al, Br J Nutr. 64, 71-79, 1990 et Arrigoni et al., Am J Clin Nutr. 60: 926-929, 1994) ont mis en évidence le fait que ces personnes digèrent et tolèrent mieux le lactose contenu dans les yogourts que celui contenu dans le lait. Cette meilleure digestion et cette meilleure tolérance au lactose sont dues notamment à l'activité de la β-galactosidase des bactéries contenues dans les yogourts lors du transit intestinal.

Par ailleurs, il est connu que le D(-)-lactate peut conduire à des problèmes d'acidose chez les enfants. Pour ces raisons, l'Organisation Mondiale de la Santé (F.A.O/O.M.S, 1967; 1974) recommande que le D(-)-lactate ne soit pas ajouté, seul ou en mélange racémique avec du L(+)-lactate à l'alimentation infantile. De plus, pour les adultes, il est préférable que la limite de la consommation de D(-)-lactate par jour ne dépasse pas 100 mg/kg du corps humain.

A ce jour, des souches bactériennes recombinées génétiquement, de manière à ce qu'elles ne produisent plus que du L(+)-lactate, sont connues.

Ainsi, T. Bhowmik et al. (Appl. Microbiol Biotechnol, 432-439, 1994) décrivent une technique d'isolation et d'inactivation par mutagénèse dirigée du gène codant pour l'enzyme D-lactate deshydrogénase de la souche de *Lactobacillus helveticus* CNRZ32 et, en particulier, la souche de *Lactobacillus helveticus* CNRZ32(pSUW104), ne produisant plus que du L(+)-lactate. Cette souche est obtenue par éléctroporation du vecteur d'intégration pSUW104, comprenant le vecteur pSA3 et le fragment interne *Sal*I*-Sph*I de 0,6 kb du gène codant pour l'enzyme D-lactate déshydrogénase de *Lactobacillus helveticus*.

Mais, à ce jour, on ne connaît pas de souches bactériennes ayant les capacités, de survie dans l'intestin, d'adhésion aux cellules intestinales et d'immunomodulation, qui ont été recombinées génétiquement, de manière qu'elles ne produisent plus que du L(+)-lactate. Or, il serait très intéressant, pour la préparation de compositions alimentaires, d'avoir de telles souches bactériennes ayant la capacité de rester vivantes dans le tractus intestinal, présentant ces propriétés bénéfiques sur la santé humaine et ne produisant plus que du L(+)-lactate, de manière à éviter les effets néfastes dûs au D(-)-lactate.

La présente invention a pour but de répondre à ces besoins.

### RESUME DE L'INVENTION:

A cet effet, la présente invention concerne une souche bactérienne ayant les capacités, de survie dans l'intestin, d'adhésion aux cellules intestinales humaines et d'immunomodulation, caractérisée par le fait qu'il s'agit d'une souche de *Lactobacillus acidophilus*, de *Lactobacillus johnsonii*, de *Lactobacillus gasseri*, de *Lactobacillus crispatus*, de *Lactobacillus amylovorus* ou de *Lactobacillus gallinarum,* qu'elle est récombinée génétiquement, de manière qu'elle ne produise plus que du L(+)-lactate, le gène codant pour l'enzyme D-lactate déshydrogénase étant inactivé.

La présente invention a également pour objet la souche CNCM-I-1851 et la souche CNCM-I-1852.

Un autre objet de la présente invention est un procédé de production d'une telle souche et l'utilisation d'une telle souche bactérienne, obtenue par la mise en oeuvre du procédé selon la présente invention, dans la préparation de compositions alimentaires.

### DESCRIPTION DETAILLEE DE L'INVENTION:

Dans la suite de la description, on emploiera l'expression "vecteur conjugatif" pour désigner un vecteur d'ADN transférable par conjugaison entre deux souches d'espèces différentes de bactéries lactiques.

Par ailleurs, dans la suite de la description, on emploiera l'expression "souche ayant la capacité de survie dans l'intestin" pour désigner une souche bactérienne lactique que l'on retrouve dans les selles, après consommation.

Enfin, dans la suite de la description on emploiera l'expression "souche bactérienne ayant la capacité d'immunomodulation" pour désigner une souche bactérienne lactique ayant un effet bénéfique sur le système immunitaire, notamment la propriété d'augmenter la phagocytose des macrophages.

La présente invention a donc pour objet une souche bactérienne ayant les capacités de survie dans l'intestion, d'adhésion aux cellules intestinales humaines et d'immunomodulation, caractérisée par le fait qu'il s'agit d'une souche de *Lactobacillus acidophilus*, de *Lactobacillus johnsonii*, de *Lactobacillus gasseri*, de, *Lactobacillus crispatus*, de *Lactobacillus amylovorus* ou de *Lactobacillus gallinarum*, qu'elle est recombinée génétiquement, de manière qu'elle ne produise plus que du L(+)-lactate, le gène codant pour l'enzyme D-lactate déshydrogénase étant inactivé.

On a isolé notamment deux souches de *Lactobacillus johnsonii* qui ont été recombinées génétiquement, de manière qu'elles ne produisent plus que du L(+)-lactate. Ces souches ont été déposées le 20/02/97, selon le traité de Budapest, à la Collection Nationale de Cultures de Microorganismes, INSTITUT PASTEUR, 25, rue du Docteur Roux, F-75724 PARIS CEDEX 15 où elles ont reçu respectivement le numéro de dépôt CNCM I-1851 et le numéro de dépôt CNCMI-1852.

La présente invention a également pour objet un procédé de préparation d'une telle souche dans lequel on isole la séquence du gène codant pour l'enzyme D-lactate deshydrogénase d'une souche bactérienne hôte ayant les capacités de survie dans l'intestin, d'adhésion aux cellules intestinales humaines et d'immunomodulation étant une souche de *Lactobacillus acidophilus*, de *Lactobacillus johnsonii*, de *Lactobacillus gasseri*, de, *Lactobacillus crispatus*, de *Lactobacillus amylovorus* ou de *Lactobacillus gallinarum*, on éffectue une mutagénèse dirigée sur cette séquence, de manière à obtenir une séquence modifiée, on intègre cette séquence modifiée dans un vecteur conjugatif, on transfère par conjugaison le vecteur conjugatif dans la souche bactérienne hôte, puis on sélectionne les bactéries hôtes dans lesquelles la séquence codant pour l'enzyme D-lactate déshydrogénase a été remplacée par recombinaison homologue avec la séquence modifiée.

Dans le procédé, selon la présente invention, on peut isoler la séquence du gène codant pour l'enzyme D-lactate déshydrogénase de la souche bactérienne hôte par PCR, par clonage ou par complémentation, par exemple.

On peut effectuer une mutagénèse dirigée sur cette séquence, de manière à obtenir une séquence modifiée, selon la méthode Gene Splicing Overlap Extension (Molecular Biotechnology, R.M. Horton, 1995, 3, 93-99) qui consiste à générer une séquence de gène dans laquelle sont introduits ou sont délétés un ou plusieurs nucléotides, par exemple.

Pour intégrer la séquence modifiée dans un vecteur conjugatif d'une souche bactérienne donneuse de la souche bactérienne hôte, on peut sélectionner une souche bactérienne donneuse contenant un vecteur conjugatif n'ayant pas la capacité de se répliquer dans la souche bactérienne hôte, on peut réaliser une construction par ligation de la séquence modifiée dans un premier vecteur incapable de se multiplier dans la souche bactérienne donneuse de la bactérie hôte, on peut introduire cette construction dans la souche bactérienne donneuse, puis on peut sélectionner les bactéries donneuses dans lesquelles le premier vecteur et le vecteur conjugatif se sont recombinés, par exemple.

On transfère donc par conjugaison le vecteur conjugatif, contenant la séquence modifiée, dans la souche bactérienne hôte.

Puis, on sélectionne les bactéries hôtes dans lesquelles la séquence codant pour l'enzyme D-lactate déshydrogénase a été remplacée par recombinaison homologue avec la séquence modifiée.

On peut sélectionner, sur un milieu contenant certaines antibiotiques, les bactéries hôtes ayant intégré le vecteur conjugatif dans leur génome, par exemple. En effet, ces bactéries, du fait de l'intégration du vecteur conjugatif dans leur génome peuvent exprimer les gènes de résistance aux antibiotiques, contenus dans la séquence dudit vecteur.

Enfin, on sélectionne les bactéries hôtes dans lesquelles les séquences d'ADN du vecteur conjugatif ont été éliminées du génome, à l'exception de la séquence modifiée.

Pour ce faire, on peut effectuer une première sélection sur un milieu contenant des antibiotiques, de manière à sélectionner les bactéries sensibles à ces antibiotiques, c'est à dire, les bactéries sauvages et les bactéries transformées génétiquement ne contenant plus que le fragment de la séquence modifiée du gène, par exemple.

Puis, on peut effectuer un test enzymatique coloré en présence de D-lactate déshydrogénase, de sel de tetrazolium et de diaphorase, de manière à différencier les bactéries sauvages des bactéries transforméées génétiquement selon la présente invention, par exemple. Ce test enzymatique permet de mettre en évidence le fait que les bactéries, ne produisant pas de D(-)-lactate, ne peuvent pas oxyder le D(-)-lactate lorsque l'on ajoute l'enzyme D-lactate déshydrogénase dans le milieu aussi, par conséquent, le sel de tetrazolium, dans le milieu, n'est pas réduit par l'enzyme diaphorase, en absence de D-lactate oxydé et ces bactéries restent incolores.

On peut alors effectuer une PCR des séquences génomiques de la bactérie hôte recombineé génétiquement, selon la présente invention, en utilisant des amorces spécifiques de la souche bactérienne hôte puis on peut effectuer une digestion du produit de PCR en présence d'enzymes de restriction spécifiques et comparer la tailles de fragments ainsi générés avec ceux obtenus, après digestion avec ces mêmes enzymes de restriction, à partir du génome d'une bactérie hôte sauvage, par exemple.

Enfin, la présente invention a pour objet l'utilisation d'une telle souche bactérienne, obtenue par la mise en oeuvre du procédé selon la présente invention, pour la préparation de compositions alimentaires.

Le procédé de préparation des souches bactériennes selon la présente invention ainsi que ces souches bactériennes recombinées génétiquement sont caractérisés plus en détails ci-après à l'aide d'analyses biochimiques et moléculaires, en référence au dessin annexé, dans lequel:
- la figure 1 représente le vecteur pLL83 qui est le produit de ligation entre le vecteur pGEMT, commercialisé par la société Promega, MADISON, WI - USA, et la séquence modifiée du gène codant pour l'enzyme D-lactate déshydrogénase,
- la figure 2 représente le vecteur pMD14, construit à partir du vecteur pBlueScript SK+ de *Escherichia coli* (Stratagene, LA YOLLA, CA - USA) et qui contient le gène de résistance au chloramphénicol (cat) du vecteur pNZ12 (Gasson et al., J. Bacteriol., 154, 1-9, 1983) et la région 5' en amont de la séquence du gène de résistance à l'érythromycine du vecteur pAMβ1 (Clewell et al., J. Bacteriol., 33, 426-428, 1974) qui a été isolée du plasmide pUC-838 (Mollet et al., J. Bacteriol., 175, 4315-4324, 1993),
- la figure 3 représente le vecteur pLL88 qui est le produit de ligation du fragment du vecteur pLL83, comprenant la séquence modifiée du gène codant pour l'enzyme D-lactate déshydrogénase, dans le vecteur pMD14,
- enfin, la figure 4 représente le vecteur pLL91, comprenant la séquence du vecteur pLL88 et celle du vecteur pAMβ1 (Clewell et al., J. Bacteriol., 33, 426-428, 1974).

### I. Isolation de la séquence du gène codant pour l'enzyme D-lactate déshydrogénase de Lactobacillus johnsonii La 1:

On fait pousser *Lactobacillus johnsonii* La 1 sur un milieu MRS pendant la nuit à 37° C. Puis, on transfère la culture dans un tube contenant un milieu MRS et on la laisse pousser jusqu'à une DO₆₀₀ d'environ 1.

On isole le génome de *Lactobacillus johnsonii* La 1 selon la méthode décrite dans l'article "DNA probe for *Lactobacillus delbrueckii*" (B. Mollet et al., Applied and Environment Microbiology, June 1990, vol 56(6), p. 1967-1970).

On isole alors la séquence du gène codant pour l'enzyme D-lactate déshydrogénase de *Lactobacillus johnsonii* La1 par PCR, en utilisant, comme amorces spécifiques, les séquences SEQ ID NO:1 et SEQ ID NO:2, décrites ci-après, qui sont des séquences de régions conservées du gène codant pour l'enzyme D-lactate déshydrogénase de *Lactobacillus helveticus* (Eur. J. Biochem., Cloning and overexpression of Lactobacillus helveticus D-lactate deshydrogenase gene in Escherichia coli, Kochhar et al., 208:799-805, 1992).

On obtient ainsi un fragment de 890 pb que l'on clone dans le vecteur pGEMT commercialisé par la société Proméga, MADISON, WI - USA et que l'on séquence ensuite. Afin d'isoler la séquence complète du gène codant pour l'enzyme D-lactate déshydrogénase de *Lactobacillus johnsonii* La1, on effectue alors un Southern blot avec différents enzymes de restriction et en utilisant comme sonde la séquence obtenue précédemment par PCR.

On isole ainsi une séquence nucléotidique de 3 kb comprenant deux cadres de lecture ouverte d'orientation opposée.

On constate une forte homologie entre un des cadres de lecture ouverte et la séquence du gène codant pour l'enzyme D-lactate déshydrogénase de *Lactobacillus helveticus*. La séquence de ce cadre de lecture ouverte a une longueur de 1014 nucléotides, ayant 85% d'homologie avec la séquence du gène codant pour l'enzyme D-lactate déshydrogénase de *Lactobacillus helveticus* et 81% d'homologie avec celle de *Lactobacillus bulgaricus* (FBES Lett., Bernard et al., 1991, 290, 61-64). Cette séquence code pour un polypeptide de 338 acides aminés.

### II. Mutagénèse dirigée sur la séquence codant pour l'enzyme D-lactate déshydrogénase de Lactobacillus johnsonii La 1:

On effectue une mutagénèse dirigée selon la méthode Gene Splicing Overlap Extension (Molecular Biotechnology, R.M. Horton, 1995, 3, 93-99) de la séquence ainsi isolée. On effectue par PCR sur cette séquence une délétion de 11 nucléotides et une insertion de 3 nucléotides au centre. Ces modifications de séquence ont pour effet de faire apparaître un site de restriction *Dra*I et de supprimer un site de restriction *EcoR*V. On utilise ces deux modifications de site de restriction, comme marqueur, pour mettre en évidence la présence de la séquence modifiée du gène codant pour la D-lactate déshydrogénase de *Lactobacillus johnsonii* La1 dans lés différents vecteurs utilisés dans la suite de la construction ainsi que dans la sélection des mutants ayant intégrés uniquement la séquence modifiée du gène codant pour l'enzyme D-lactate déshydrogénase.

La séquence ainsi modifiée du gène code pour un polypeptide de 181 acides aminés au lieu d'un polypeptide 338 acides aminés.

### III. Clonage de la séquence modifiée du gène codant pour l'enzyme D-lactate déshydrogénase de Lactobacillus johnsonii Lal dans le vecteur pGEMT de Escherichia coli:

On clone la séquence modifiée du gène codant pour l'enzyme D-lactate déshydrogénase dans le vecteur pGEMT d'*Escherichia coli*.

Pour ce faire, on ligue la séquence modifiée du gène dans ce vecteur pGEMT contenant le gène de resistance à l'ampicilline.

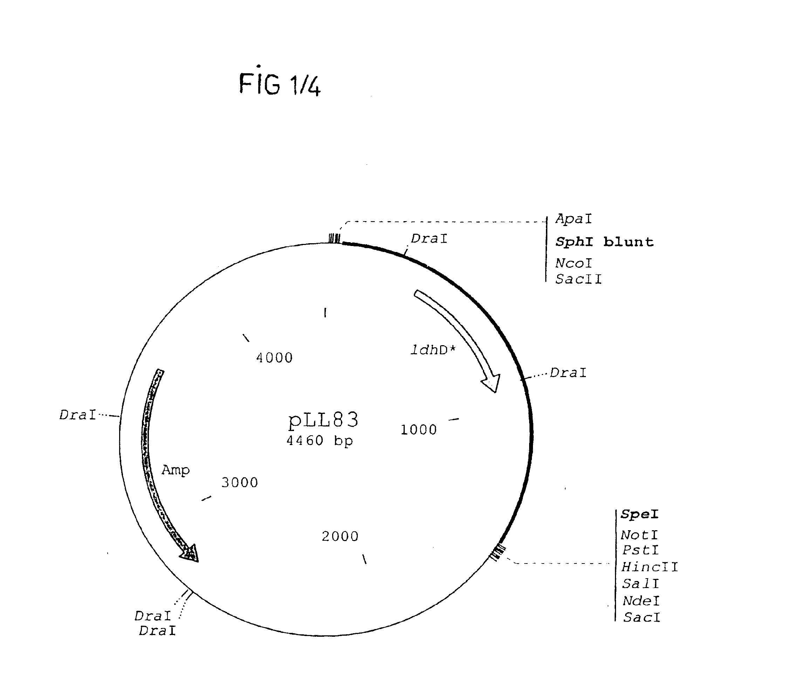

On introduit ensuite ce mélange de ligation dans *Escherichia coli* XL1- Blue par électroporation et l'on sélectionne les clones positifs en présence de X-gal et d'IPTG (Sambrook et al., Molecular cloning:a laboratory manuel, 2nd ed., 1989). On dénome le vecteur ainsi obtenu, tel que repérsenté à la figure 1, pLL83.

Puis, on purifie le vecteur pLL83 selon la méthode de la lyse alcaline (Sambrook et al., Molecular cloning:a laboratory manuel, 2nd ed., 1989).

On isole alors du vecteur pLL83, ainsi purifié, le fragment, comprenant la séquence modifiée du gène codant pour l'enzyme D-lactate déshydrogénase. Pour ce faire, on effectue une digestion avec l'enzyme de restriction *Sph*I, au niveau du site de restriction *Sph*I sur le vecteur pLL83. On fait ensuite réagir l'enzyme T4 polymérase au niveau de cette coupure, de manière à ajouter des nucléotides et obtenir une coupure franche. Enfin, on effectue une digestion avec l'enzyme de restriction *Spe*I.

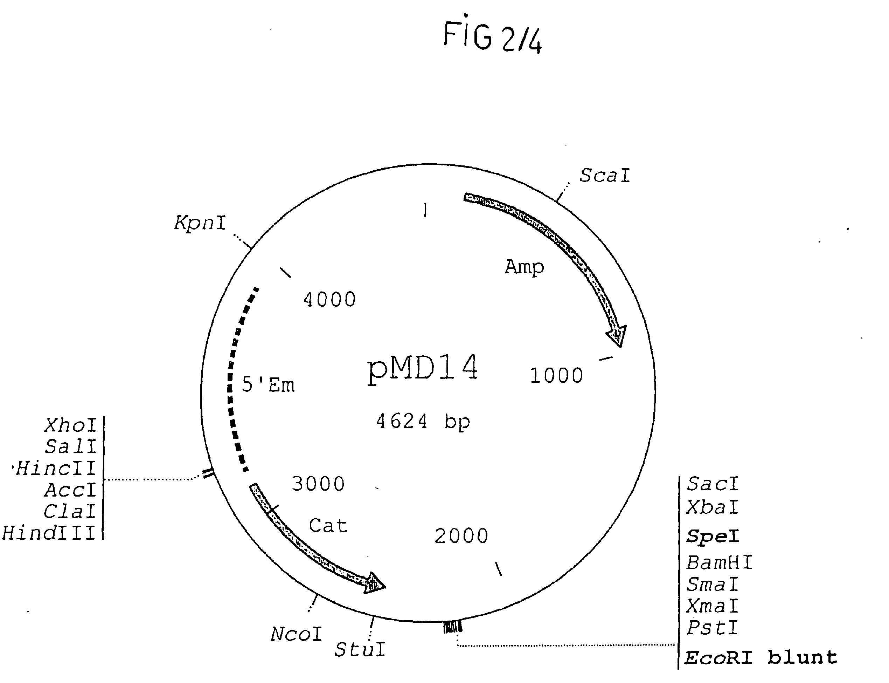

Parallèlement, on effectue une digestion sur un vecteurqui est incapable de se répliquer dans la souche bactérienne donneuse, *Lactococcus lactis* et dans la souche bactérienne hôte, *Lactobacillus johnsonii*. On effectue cette digestion au niveau d'un site de restriction puis l'on fait réagir l'enzyme T4 polymérase au niveau de cette coupure, de manière à ajouter des nucléotides et obtenir une coupure franche. Enfin, on effectue une digestion avec l'enzyme de restriction *Spe*I. On peut notamment utiliser le vecteur pMD 14, décrit à la figure 2, pour réaliser cette construction. On peut effectuer une digestion sur ce vecteur pMD14 avec l'enzyme de restriction *Eco*RI, puis faire réagir l'enzyme T4 polymérase et enfin effectuer une digestion avec l'enzyme de restriction *Spe*I.

On introduit alors le fragment du vecteur pLL83, comprenant la séquence modifiée du gène codant pour l'enzyme D-lactate déshydrogénase, dans le vecteur pMD14.

On introduit alors le mélange de ligation dans *Escherichia coli* XL1-Blue par électroporation.

On dépose ensuite une centaine de colonies de *Escherichia coli* Xl1-Blue ainsi construites sur des plaques de microfiltration, on les transfère alors sur une membrane de nitrocellulose, puis on les lyse *in situ* et l'on effectue une hybridation avec un fragment interne du gène codant pour l'enzyme D-lactate déshydrogénase de *Lactobacillus johnsonii* La1.

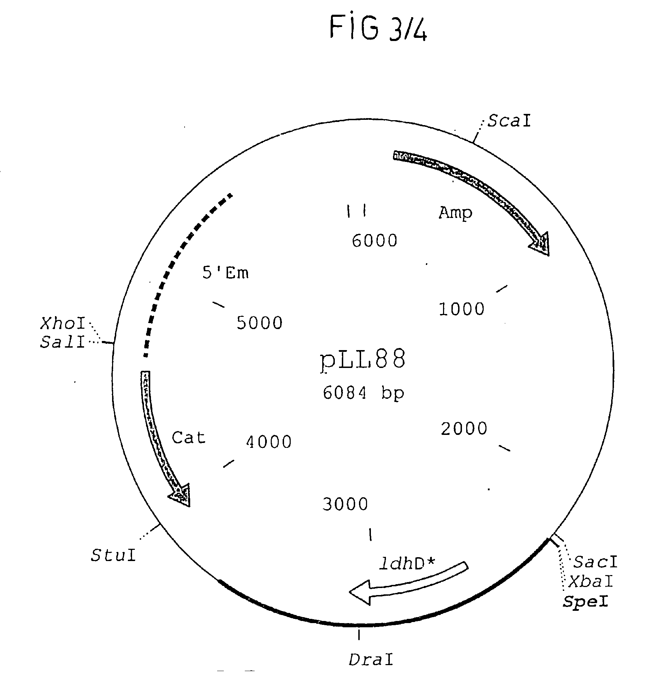

On selectionne ainsi 3 clones positifs, contenant le vecteur pLL88, décrit à la figure 3. Le séquençage du vecteur pLL88 met en évidence, le fait que ce dernier comprend la séquence modifiée du gène codant pour l'enzyme D-lactate déshydrogénase de *Lactobacillus johnsonii* La1, les régions de pGEMT adjacentes à cette séquence et le vecteur pMD14.

### IV. Introduction du vecteur pLL88 dans la souche Lactococcus lactis MG1363(pAMβ1):

On introduit le vecteur pLL88 dans la souche *Lactococcus lactis* MG1363(pAMβ 1), par électroporation.

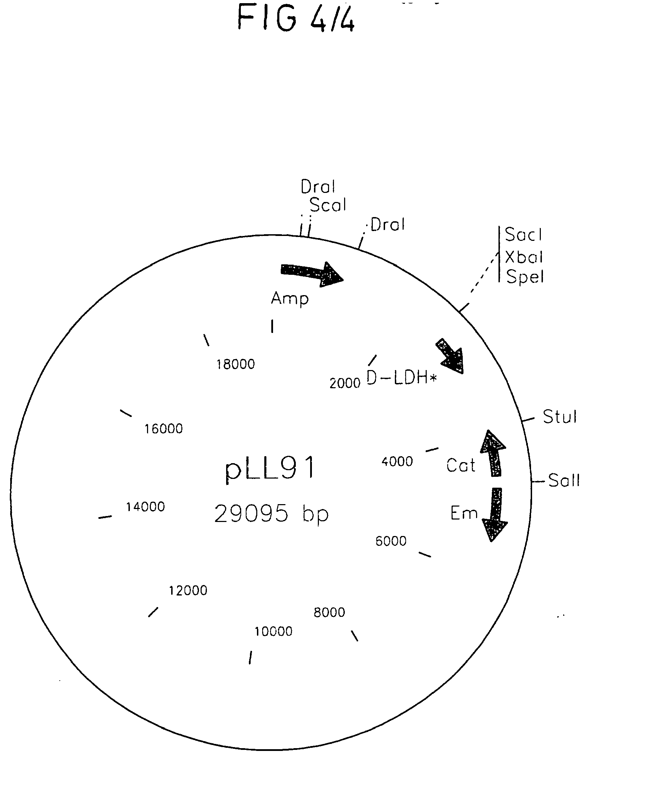

On place ensuite les transformants ainsi réalisés sur un milieu agar GM17, contenant 12 µg/ml de chloramphénicol et l'on incube à 30° C pendant toute une nuit. Le vecteur pLL88 ne peut pas se repliquer dans les bactéries Gram positif. Oon sélectionne ainsi, sur ce milieu contenant du chloramphénicol, toutes les bactéries *Lactococcus lactis* MG1363(pAMβ1) ayant intégré, par région homologue, dans le vecteur conjugatif pAMβ1, décrit à la figure 5, le vecteur pLL88, comprenant le gène de résistance au chloramphénicol. On dénommera par la suite cette construction, comprenant la séquence du vecteur pLL88 et celle du vecteur pAMβ1, le vecteur pLL91, décrit à la figure 4.

### V. Conditions de conjugaison:

On cultive *Lactococcus lactis* MG1363, contenant le vecteur pLL91 sur un milieu GM17 et 12 µg/ml de chloramphénicol et *Lactobacillus johnsonii* La1 sur milieu MRS.

On inocule 0,2% de la culture de *Lactobacillus johnsonii* La1 ainsi préparé dans un tube contenant un milieu frais de MRS et on laisse incuber pendant 5h00 à 37° C.

On centrifuge alors la culture de *Lactococcus lactis* MG1363 et celle de *Lactobacillus johnsonii* La1 à 3000 rpm pendant 5 min et l'on transfère chaque culot dans 10 ml de milieu LCMG (Efthymiou et al., An antigenic analysis of lactobacillus acidophilus J., Infect. Dis., 1962, 110: 258-267), contenant 10 g de trypticase, 5 g d'extrait de levure, 3 g de tryptose, 3g de K₂HPO₄, 3 g de KH₂PO₄, 2 g de citrate d'ammonium, 5 ml de solution enrichie en sels minéraux, 1 g de Tween 80, 1 g d'acétate de sodium, 20 g de glucose et 0,2 g de cystéine.

Puis, on mélange 1 ml de culture de la souche donneuse *Lactococcus lactis* MG1363, ainsi préparée, avec 10 ml de culture de la souche receveuse *Lactobacillus johnsonii* La1, ainsi préparée, et l'on centrifuge le mélange.

On écarte le surnageant et l'on dépose le culot ainsi concentré sur des plaques de milieu PEG-agar (Takemoto et al., Agric. Biol. Chem., 1989, 53:3333-3334), contenant 5 g de PEG6000, 15 g d'agar, 1000 ml de solution LCMG sans sucre, 100 ml de solution sucrée et 10 ml de solution de sels minéraux. On dispose ces plaques à température ambiante jusqu'à ce que le culot soit sec et on le recouvre alors de 10 ml de milieu LCMG contenant 7‰ d'agar.

On incube alors ces plaques toute une nuit à 37° C, avant de découper l'agar contenant les cellules bactériennes ayant poussé et de déposer cet agar dans des tubes contenant 10 ml de milieu LCMG.

On mélange alors vigoureusement ces tube et on dilue les cultures ainsi préparées dans du milieu TS, contenant 1 g/l de tryptone et 8,5 g/l de NaCl.

On dépose alors les cultures diluées sur des plaques de milieu MRS-agar, contenant 100 µg/ml de phosphomycine et 14 µg/ml de chloramphénicol et on laisse incuber à 37° C pendant 48h00 en conditions anaérobes.

### VI. Conjugaison et intégration dans Lactobacillus johnsonii La1 du vecteur pLL91:

On effectue la conjugaison de *Lactococcus lactis* MG1363, contenant le vecteur pLL91, avec *Lactobacillus johnsonii* La1. Cette conjugaison a une fréquence comprise entre 1.10⁻⁵ et 3.10⁻⁷ transformants/cellules receveuses.

On sélectionne alors 6 colonies de *Lactobacillus johnsonii* La1 résistantes au chloramphénicol et à la phosphomycine que l'on cultive alors à 37° C sur un milieu MRS, avant de les transférer quelques heures à 45° C, de manière à sélectionner les bactéries *Lactobacillus johnsonii* La1 ayant intégré le vecteur pLL91 dans leur génome, par région homologue, au niveau de la séquence codant pour l'enzyme D-lactate déshydrogénase. En effet, le vecteur pAMβ1, contenu dans le vecteur pLL91, est incapable de se répliquer à une température supérieure à 42° C. Ainsi, toutes les bactéries *Lactobacillus johnsonii* La1 résistante au chloramphénicol et pouvant pousser à 45° C ont intégré le vecteur pLL91 dans leur génome, par région homologue, au niveau de la séquence codant pour l'enzyme D-lactate déshydrogénase.

Aussi, on obtient l'intégration du vecteur pLL91 dans le génome de *Lactobacillus johnsonii* La1, soit par simple crossing over au niveau de la région 5' terminale de la séquence du gène codant pour la D-lactate déshydrogénase, soit au niveau de la région 3' terminale de la séquence de ce gène.

### VII. Vérification de l'intégration de la séquence modifiée du gène codant pour l'enzyme D-lactate déshydrogénase sur le DNA chromosomique de Lactobacillus johnsonii La1:

On vérifie par PCR l'intégration du vecteur pLL91 dans le génome de *Lactobacillus johnsonii* La1.

Pour ce faire, on utilise des amorces spécifiques du génome de *Lactobacillus johnsonii* La1, ayant comme séquences les séquences SEQ ID NO:3 et SEQ ID NO:4, décrites ci-après, ainsi que des amorces spécifiques du vecteur pLL91, ayant comme séquence les séquences SEQ ID NO:5 et SEQ ID NO:6, décrites ci-après. Puis l'on digère les fragments ainsi amplifiés par PCR avec l'enzyme de restriction *Eco*RV, dont le site de restriction se situe dans la séquence originelle du gène codant pour l'enzyme D-lactate déshydrogénase, et avec l'enzyme de restriction *Dra*I, dont le site de restriction se situe dans la séquence modifiée du gène codant pour l'enzyme D-lactate déshydrogénase, de manière à mettre en évidence le fait que l'intégration par simple crossing-over s'est fait au niveau de la région 5' terminale ou au niveau de la région 3' terminale de la séquence originelle du gène. On sélectionne alors une bactérie *Lactobacillus johnsonii* La 1 modifiée génétiquement par intégration de la séquence modifiée du gène dans la région 5' terminale de la séquence originelle du gène et une bactérie *Lactobacillus johnsonii* La1 modifiée génétiquement par intégration de la séquence modifiée du gène dans la région 3' terminale de la séquence originelle du gène.

On vérifie ensuite l'intégration du vecteur pLL91 dans le génome de *Lactobacillus johnsonii* La1, en réalisant un Southern blot du génome de ces deux bactéries *Lactobacillus johnsonii* La1 modifiée génétiquement, sélectionnées ci-dessus. Pour ce faire, on digère le DNA génomique de ces deux bactéries avec différents enzymes de restriction, ayant leurs sites de restriction situés dans le génome, sur le vecteur pLL91 et sur la séquence modifiée du gène codant pour la D-lactate déshydrogénase. On utilise comme sonde d'hybridation un fragment de la séquence originelle du gène codant pour l'enzyme D-lactate déshydrogénase.

On met ainsi en évidence le fait que les fragments de génome des deux bactéries *Lactobacillus johnsonii* La1 modifiées génétiquement, obtenus après digestion avec différents enzymes de restriction, sont de taille différente par rapport à ceux obtenus après digestion avec ces mêmes enzymes de restriction du génome de la bactérie *Lactobacillus johnsonii* La1 sauvage.

### VIII. Résolution de l'intégration:

La résolution de l'intégration se fait par libération du vecteur pLL91 hors du génome. On obtient la perte du vecteur pLL91 soit par simple crossing-over entre la région 5' terminale de la séquence originelle du gène codant pour l'enzyme D-lactate déshydrogénase et celle de la séquence modifiée dudit gène, soit par simple crossing-over entre la région 3' terminale de la séquence originelle du gène codant pour l'enzyme D-lactate déshydrogénase et celle de la séquence modifiée dudit gène.

On effectue la résolution de l'intégration à 37° C, qui est une température permissive pour le vecteur, de manière à favoriser la libération du vecteur pLL91 hors du génome.

On effectue un test enzymatique coloré en présence de D-lactate déshydrogénase, de sel de tetrazolium et de diaphorase, de manière à différencier les bactéries sauvages des bactéries transforméées génétiquement selon la présente invention. Ce test enzymatique permet de mettre en évidence le fait que les bactéries, ne produisant pas de D(-)-lactate, ne peuvent pas oxyder le D(-)-lactate lorsque l'on ajoute l'enzyme D-lactate déshydrogénase dans le milieu aussi, par conséquent, le sel de tetrazolium, dans le milieu, n'est pas réduit par l'enzyme diaphorase, en absence de D-lactate oxydé et ces bactéries restent incolores.

Les exemples ci-après sont donnés à titre d'illustration de l'utilisation d'une souche bactérienne selon la présente invention pour la fabrication de compositions alimentaires. Les pourcentages sont donnés en poids, sauf indication contraire.

### Exemple 1

On prépare des yogourts à partir de la souche de *Lactobacillus johnsonii* CNCM I-1851, obtenue par la mise en oeuvre du procédé selon la présente invention.

Pour ce faire, on prépare 500 ml de lait écrémé en poudre reconstitué à 9%, on y ajoute 0,1% d'extrait de levure et on stérilise à l'autoclave pendant 15 min à 121° C. Puis, on laisse refroidir à 40° C avant d'y incorporer 10% en volume d'une culture active de la souche *Lactobacillus johnsonii* CNCM I-1851, contenant 5.10⁸ de microorganismes/cm³.

On laisse incuber cette préparation pendant 4h00 à 40° C, de manière à réaliser un levain contenant environ 2,5.10⁸ de microorganismes/cm³.

Parallèlement, on prépare, selon la méthode décrite ci-dessus, un levain contenant environ 5.10⁸ de *Streptococcus thermophilus* épaississante/cm³.

On pasteurise à 90° C pendant 30 min un mélange contenant 1,5% de matière grasse, 3% de lait écrémé en poudre. Puis, on ajoute à ce mélange 1% de levain de *Lactobacillus johnsonii* CNCM I-1851 et 3% de levain de *Streptococcus thermophilus*.

On mélange ensuite cette préparation et on la laisse incuber pendant 4h20 à 40° C, de manière à obtenir une préparation ayant un pH de 4,6.

On obtient ainsi des yogourts à la texture agréable, dont la concentration de *Lactobacillus johnsonii* CNCM I-1851 est de 1.10⁸ cellules/cm³ et celle de *Streptococcus thermophilus* est de 1.10⁸ cellules/cm³.

### Exemple 2

On procède de la manière telle que décrite à l'exemple 1, à l'exception du fait que l'on dilue à 50% les yogourts ainsi réalisés avec de l'eau distillée stérile, de manière à pouvoir utiliser cette préparation en alimentation parentérale, en milieu hospitalier.

### Exemple 3

On prépare un lait fermenté à partir de la souche de *Lactobacillus johnsonii* CNCM I-1852, obtenue par la mise en oeuvre du procédé selon la présente invention.

Pour ce faire, on chauffe 1 l de lait à 120° C pendant 15 min, de manière à le dénaturer.

Puis, on le refroidit à 37° C et on l'inocule avec 5% v/v de *Lactobacillus johnsonii* CNCM I-1852, obtenu par la mise en oeuvre du procédé selon la présente invention.

On laisse incuber la préparation ainsi réalisée à température ambiante pendant 18 à 24h, jusqu'à ce que son taux d'acidité atteigne une valeur de 1%.

Enfin on conditionne le lait fermenté ainsi réalisé en bouteille que l'on stocke à température réfrigérée.

### Exemple 4

On prépare un fromage frais à partir de la souche de *Lactobacillus johnsonii* CNCM I-1852, obtenue par la mise en oeuvre du procédé selon la présente invention.

Pour ce faire, on chauffe 1 1 de lait à 72° C pendant 15 min, puis on le laisse refroidir à 19° C.

On l'inocule alors avec 0,5% v/v d'un mélange bactérien, contenant un *Lactococcus lactis cremoris,* un *Lactococcus lactis diacetylactis* et la souche *Lactobacillus johnsonii* CNCM I-1852.

On laisse incuber à environ 20° C, jusqu'à ce que la valeur du pH du lait soit de 4,6.

On verse ensuite le lait ainsi coagulé dans des sachets en nylon, de manière à faire écouler l'excédent d'eau contenu dans le fromage frais, ainsi réalisé.

Puis, on mélange le fromage frais avec un agent antimycotique, tel que le sorbate de potassium, de manière à prévenir la moisissure.

Enfin, on l'homogénéise en le mélangeant lentement, de manière à obtenir un fromage frais à la texture lisse.

Le fromage frais, aini réalisé est conditionné dans des petits pots qui peuvent être conservés à 4-5° C pendant 4 à 5 semaines.

## Revendications

1. Souche bactérienne ayant les capacités de survie dans **caractérisée par le fait qu'**il s'agit d'une souche de *Lactobacillus acidophilus*, de *Lactobacillus johnsonii*, de *Lactobacillus gasseri*, de *Lactobacillus crispatus*, de *Lactobacillus amylovorus* ou de *Lactobacillus gallinarum* et l'intestin, d'adhésion aux cellules intestinales humaines et d'immunomodulation, **par le fait qu'**elle est recombinée génétiquement, de sorte qu'elle ne produise plus que du L(+)-lactate, le gène codant pour l'enzyme D-lactate déshydrogénase étant inactivé.

2. Souche selon la revendication 1, déposée à la CNCM sous le numéro I-1851.

3. Souche selon la revendication 1, déposée à la CNCM sous le numéro I-1852.

4. Procédé de production d'une souche selon la revendication 1 dans lequel:
- on isole la séquence du gène codant pour la D-lactate deshydrogénase d'une souche bactérienne hôte ayant les capacités de survie dans l'intestin, d'adhésion aux cellules intestinales humaines et d'immunomodulation,
- on effectue une mutagénèse dirigée sur cette séquence, de manière à obtenir une séquence modifiée,
- on intègre cette séquence modifiée dans un vecteur conjugatif,
- on transfère par conjugaison le vecteur conjugatif dans la souche bactérienne hôte,
- puis on sélectionne les bactéries hôtes dans lesquelles la séquence codant pour l'enzyme D-lactate déshydrogénase a été remplacée par recombinaison homologue avec la séquence modifiée.

5. Procédé selon la revendication 4, dans lequel pour intégrer la séquence modifiée dans un vecteur conjugatif,
- on sélectionne une souche bactérienne donneuse contenant un vecteur conjugatif n'ayant pas la capacité de se répliquer dans la souche bactérienne hôte,
- on réalise une construction par ligation de la séquence modifiée dans un premier vecteur incapable de se multiplier dans la souche donneuse,
- on introduit cette construction dans la souche bactérienne donneuse,
- puis on sélectionne les bactéries donneuses dans lesquelles le premier vecteur et le vecteur conjugatif se sont recombinés.

6. Procédé selon la revendication 4, dans lequel on sélectionne les bactéries hôtes dans lesquelles les séquences d'ADN du vecteur conjugatif ont été éliminées du génome, à l'exception de la séquence modifiée.

7. Utilisation d'une souche bactérienne obtenue par la mise en oeuvre du procédé selon l'une des revendications 4 à 6, dans la préparation d'une composition alimentaire.

## Claims

1. A bacterial strain having the ability to survive in the intestine, to adhere to human intestinal cells and having an immunomodulatory capacity, **characterised in that** it comprises a strain of *Lactobacillus acidophilus, Lactobacillus johnsonii, Lactobacillus gasseri, Lactobacillus crispatus, Lactobacillus amylovorus* or *Lactobacillus gallinarum* and **in that** it is genetically recombined such that it now produces only L(+)-lactate, the gene encoding the enzyme D-lactate dehydrogenase being inactivated.

2. A strain according to claim 1, deposited at CNCM under number 1-1851.

3. A strain according to claim 1, deposited at CNCM under number 1-1852.

4. A method for producing a strain according to claim 1 in which:
- the sequence of the gene encoding D-lactate dehydrogenase is isolated from a host bacterial strain having the ability to survive in the intestine, to adhere to human intestinal cells and having an immunomodulatory capacity,
- directed mutagenesis is carried out on said sequence so as to obtain a modified sequence,
- this modified sequence is incorporated into a conjugative vector,
- the conjugative vector is conjugatively transferred into the host bacterial strain,
- then the host bacteria are selected in which the sequence encoding the enzyme D-lactate dehydrogenase has been replaced with the modified sequence by homologous recombination.

5. A method according to claim 4, in which, in order to incorporate the modified sequence into a conjugative vector,
- a donor bacterial strain is selected which contains a conjugative vector which does not have the ability to replicate in the host bacterial strain,
- a structure is created by ligation of the modified sequence into a first vector which is incapable of multiplying in the donor strain,
- said structure is introduced into the donor bacterial strain,
- then the donor bacteria are selected in which the first vector and the conjugative vector have recombined.

6. A method according to claim 4, in which the host bacteria selected are those in which the DNA sequences of the conjugative vector have been removed from the genome, with the exception of the modified sequence.

7. Use of a bacterial strain obtained by carrying out the method according to anyone of claims 4 to 6 in the preparation of a foodstuff composition.

## Patentansprüche

1. Bakterienstamm mit den Fähigkeiten des Überlebens im Darm, des Haftens an den menschlichen Darmzellen und zur Immunmodulation, **dadurch gekennzeichnet, dass** es sich um einen Stamm von *Lactobacillus acidophilus, Lactobacillus johnsonii, Lactobacillus gasseri, Lactobacillus crispatus, Lactobacillus amylovorus oder Lachtobacillus gallinarum* handelt und dass er genetisch so rekombiniert ist, dass er nur mehr L(+)-Lactat erzeugt, indem das für das Enzym D-Lactat-Dehydrogenase codierende Gen inaktiviert ist.

2. Stamm nach Anspruch 1, der bei der CNCM unter der Nummer 1-1851 hinterlegt ist.

3. Stamm nach Anspruch 1, der bei der CNCM unter der Nummer I-1852 hinterlegt ist.

4. Verfahren zur Herstellung eines Stamms nach Anspruch 1, bei dem:
- man die Sequenz des für die D-Lactat-Dehydrogenase codierenden Gens aus einem Wirtsbakterienstamm isoliert, der die Fähigkeiten des Überlebens im Darm, des Haftens an den menschlichen Darmzellen und zur Immunmodulation besitzt,
- man eine gerichtete Mutagenese auf dieser Sequenz so durchführt, dass man eine modifizierte Sequenz erhält,
- man diese modifizierte Sequenz in einen konjugativen Vektor integriert,
- man den konjugativen Vektor durch Konjunktion in den Wirtsbakterienstamm transferiert,
- und man dann diejenigen Wirtsbakterien selektiert, in denen die für das Enzym D-Lactat-Dehydrogenase codierende Sequenz durch homologe Rekombination mit der modifizierten Sequenz ersetzt wurde.

5. Verfahren nach Anspruch 4, bei dem man, um die modifizierte Sequenz in einen konjugativen Vektor zu integrieren,
- einen Spenderbakterienstamm selektiert, der einen konjugativen Vektor enthält, der nicht die Fähigkeit besitzt, sich in dem Wirtsbakterienstamm zu replizieren,
- man ein Konstrukt der modifizierten Sequenz durch Ligation in einem ersten Vektor herstellt, der nicht in der Lage ist, sich in dem Spenderstamm zu vermehren,
- man dieses Konstrukt in den Spenderbakterienstamm einführt,
- und man dann diejenigen Spenderbakterien selektiert, in denen sich der erste Vektor und der konjugative Vektor rekombiniert haben.

6. Verfahren nach Anspruch 4, bei dem man die Wirtsbakterien selektiert, in denen die DNA-Sequenzen des konjugativen Vektors mit Ausnahme der modifizierten Sequenz aus dem Genom entfernt wurden.

7. Verwendung eines Bakterienstamms, der mit Hilfe des Verfahrens nach einem der Ansprüche 4 bis 6 erhalten wurde, bei der Herstellung einer Nahrungsmittelszusammensetzung.


### LISTE DE SEQUENCES

(1) INFORMATION GENERALE:
   (i) DEPOSANT:
      (A) NOM: SOCIETE DES PRODUITS NESTLE
      (B) RUE: AVENUE NESTLE 55,
      (C) VILLE: VEVEY
      (D) PROVINCE: CANTON DE VAUD
      (E) PAYS: SWITZERLAND
      (F) CODE POSTAL: 1800
      (G) TELEPHONE: 021 924 34 20
      (H) TELECOPIE: 021 924 28 80
   (ii) TITRE DE L' INVENTION: PRODUCTION DE L(+)-LACTATE
   (iii) NOMBRE DE SEQUENCES: 6
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 22 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   GCTTACGCTA TTCGAAAAGA CG
   22
(2) INFORMATION POUR LA SEQ ID NO: 2:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   GTAGTGTAGA AGGCGGTGTG TGG
   23
(2) INFORMATION POUR LA SEQ ID NO: 3:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3:
   TGGTTGCCAA GTATTAG
   17
(2) INFORMATION POUR LA SEQ ID NO: 4:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   GCTAAGTCAT TAGTGCC
   17
(2) INFORMATION POUR LA SEQ ID NO: 5:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   ACAAAAGCTG GAGCTCC
   17
(2) INFORMATION POUR LA SEQ ID NO: 6:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   TTGACGTTGA GCCTCGG
   17